# EUROPEAN PATENT APPLICATION

(11) **EP 4 736 744 A1**
(43) Date of publication of application: **06.05.2026**
(21) Application number: 24860082.7
(22) Date of filing: 13.06.2024
(51) Int. Cl.: A61B 5/00, A61B 5/024, G06F 1/16

(54) **WEARABLE DEVICE COMPRISING SENSOR**

(30) Priority: 25.08.2023 KR 20230112364; 12.09.2023 KR 20230121417
(71) Applicant: Samsung Electronics Co., Ltd., Suwon-si, Gyeonggi-do 16677 (KR)
(72) Inventor: LEE, Seungwon, Suwon-si Gyeonggi-do 16677 (KR); LIM, Sukwang, Suwon-si Gyeonggi-do 16677 (KR); KWON, Hyoujoo, Suwon-si Gyeonggi-do 16677 (KR); PARK, Jaehyuck, Suwon-si Gyeonggi-do 16677 (KR); PARK, Hyuncheol, Suwon-si Gyeonggi-do 16677 (KR); YOO, Soohan, Suwon-si Gyeonggi-do 16677 (KR); JUNG, Hyunjun, Suwon-si Gyeonggi-do 16677 (KR); JO, Seongwook, Suwon-si Gyeonggi-do 16677 (KR)
(74) Representative: Appleyard Lees IP LLP
(86) International application number: PCT/KR2024/008162
(87) International publication number: WO 2025/048158

(57) **Abstract**

One embodiment can include a first ring and a second ring detachably coupled with the first ring. A first sensor in the first ring can include a light-emitting unit for emitting light toward a part of the body of user on which a wearable device is worn. A second sensor in the second ring can include a light-receiving unit for receiving at least some of the light that was emitted from the light-emitting unit and passed through the part of the body of the user through a second housing coupled to a first housing.

## Description

### [Technical Field]

Embodiments to be described later relate to a wearable device including a sensor.

### [Background Art]

A wearable device may be used in a state of being worn on a portion of a body of a user. The wearable device may be provided as various types of products. For example, the wearable device may include a ring shaped device for the user to be worn on the portion of the body of the user. The wearable device may include various electronic components. In order to meet needs of the user, the wearable device may include a sensor configured to provide information related to the user.

The above-described information may be provided as a related art for the purpose of helping understanding of the present disclosure. No argument or decision is made as to whether any of the above description may be applied as a prior art related to the present disclosure.

### [Disclosure]

### [Technical Solution]

According to an embodiment, a wearable device may comprise a first ring including a first housing including a first magnet and a first sensor in the first housing. The wearable device may comprise a second ring including a second housing including a second magnet, and a second sensor in the second housing, the second ring being detachably coupled with the first ring by magnetic force between the first magnet and the second magnet. The first sensor may include a light emitting portion configured to emit light toward a portion of a body of a user on which the wearable device is worn. The second sensor may include a light receiving portion configured to receive, via the second housing coupled to the first housing, at least a portion of the light being emitted from the light emitting portion and passing through the portion of the body of the user.

According to an embodiment, a wearable device may comprise a first ring including a first housing including a first magnet, and electronic components including a first sensor in the first housing. The wearable device may comprise a second ring including a second housing including a second magnet, and electronic components including a second sensor in the second housing, the second ring being detachably coupled with the first ring by magnetic force between the first magnet and the second magnet. The wearable device may comprise at least one processor, a Hall sensor configured to sense the magnetic force between the first magnet and the second magnet, and communication circuitry for communication with an external electronic device. The first sensor may include a light emitting portion configured to emit light toward a portion of a body of a user on which the wearable device is worn. The second sensor may include a light receiving portion configured to receive, via the second housing coupled to the first housing, at least a portion of the light being emitted from the light emitting portion and passing through the portion of the body of the user. The at least one processor may be configured to identify whether the first ring and the second ring are coupled through the Hall sensor. The at least one processor may be configured to, based on identifying that the first ring and the second ring are coupled, provide information related to the first ring and the second ring via the external electronic device connected with the wearable device by the communication circuitry.

### [Description of the Drawings]

FIG. 1 is a block diagram of an electronic device in a network environment according to an embodiment.
FIG. 2 illustrates an exemplary wearable device.
FIG. 3A illustrates a first ring of an exemplary wearable device.
FIG. 3B illustrates a second ring of an exemplary wearable device.
FIG. 3C illustrates a state in which a first ring and a second ring of an exemplary wearable device are separated.
FIG. 3D illustrates a state in which a first ring and a second ring of an exemplary wearable device are coupled.
FIG. 4 illustrates an exemplary wearable device.
FIGS. 5A and 5B are flow charts indicating an operation of a processor of an exemplary wearable device.
FIGS. 6A and 6B illustrate an exemplary wearable device.
FIG. 7 is a flow chart indicating an operation of a processor of an exemplary wearable device.
FIG. 8 illustrates an exemplary wearable device.
FIG. 9 illustrates an exemplary wearable device worn by a user.

### [Mode for Invention]

Hereinafter, an embodiment of the present disclosure is described in detail with reference to the drawings such that those having ordinary knowledge in the art to which the present disclosure belongs, may easily implement it. However, the present disclosure may be implemented in various different forms and is not limited to an embodiment described herein. Regarding descriptions of the drawings, the same or similar reference numerals may be used for the same or similar components. In addition, in the drawings and related descriptions, descriptions for well-known functions and configurations may be omitted for clarity and conciseness.

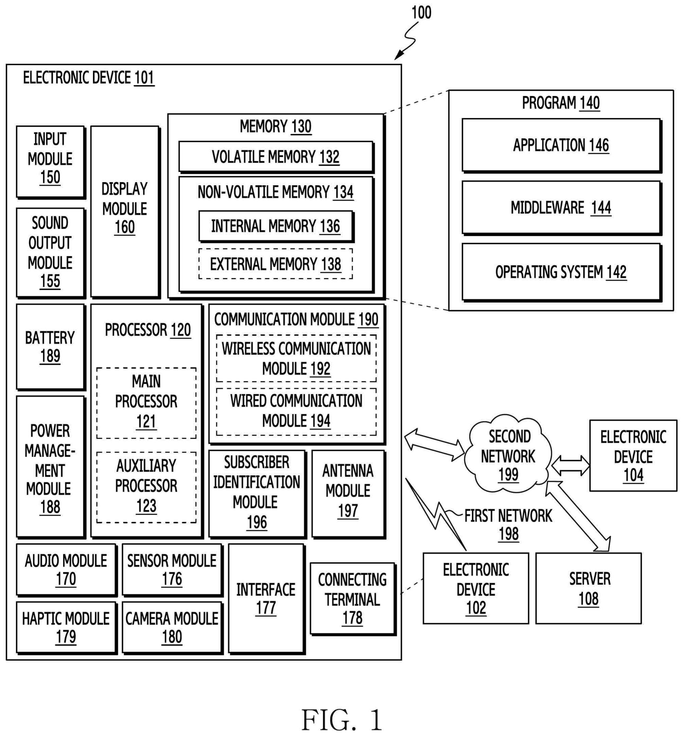

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

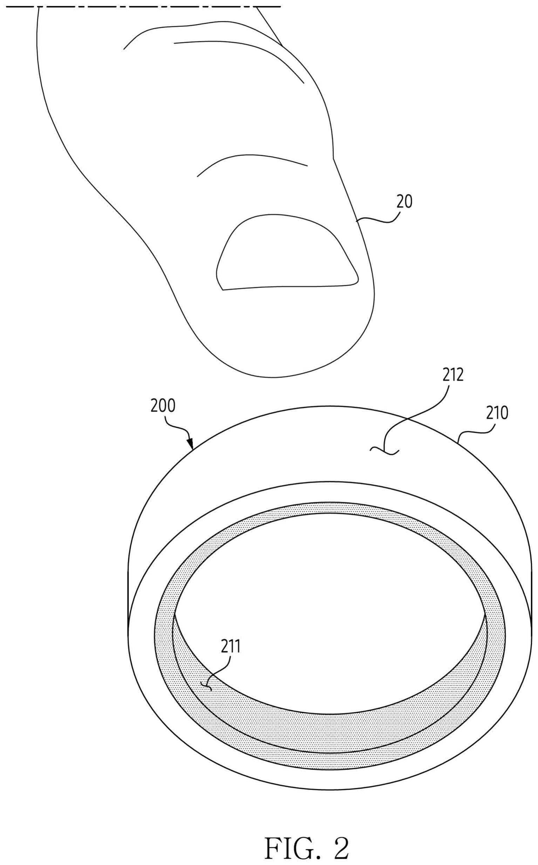

FIG. 2 illustrates an exemplary wearable device.

Referring to FIG. 2, a wearable device 200 may include a housing 210.

According to an embodiment, the wearable device 200 may be worn by a user. The user may mean a person wearing the wearable device 200. The wearable device 200 may be worn on a portion 20 of a body of the user. For example, the wearable device 200 may be worn on the portion 20 of the body of the user. For example, the wearable device 200 may be fastened to the portion 20 of the body of the user. For example, the wearable device 200 may be detachable from the portion 20 of the body of the user.

For example, the wearable device 200 may be in contact with the portion 20 of the body of the user by being worn by the user. For example, the wearable device 200 may be configured to obtain information related to the user via the portion 20 of the body of the user by being worn by the user. For example, the wearable device 200 may provide the user with information indicating a state of the user based on obtaining the information related to the user. For example, the wearable device 200 may provide the user with the information indicating the state of the user, by being configured to display the information indicating the state of the user via a display module (not illustrated) of the wearable device 200 and/or an electronic device (e.g., the electronic device 101 of FIG. 1) connected with the wearable device 200. The wearable device 200 may be referred to as the electronic device 102 or the electronic device 104 of FIG. 1 in terms of providing the user with the information related to the user wearing the wearable device 200 via the electronic device 101 connected with the wearable device 200, but is not limited thereto.

For example, the portion 20 of the body of the user on which the wearable device 200 is worn may be a finger of the user. For example, the housing 210 of the wearable device 200 may have a ring shape so that the wearable device 200 is worn on the finger of the user. However, it is not limited thereto. The wearable device 200, which may be referred to as a wearable device, may have a shape corresponding to the portion 20 of the body in order to be worn on the portion 20 of the body of the user.

According to an embodiment, the housing 210 may include an inner side 211 facing the portion 20 of the body of the user when the wearable device 200 is worn by the user. For example, in a case that the wearable device 200 is worn by the user, the inner side 211 may be at least partially in contact with the portion 20 of the body of the user. For example, the inner side 211 may surround the portion 20 of the body of the user on which the wearable device 200 is worn. For example, the inner side 211 may cover the portion 20 of the body of the user on which the wearable device 200 is worn. For example, the inner side 211 may be configured so that the wearable device 200 is fastened to the portion 20 of the body of the user, by pressurizing the portion 20 of the body of the user when the wearable device 200 is worn by the user.

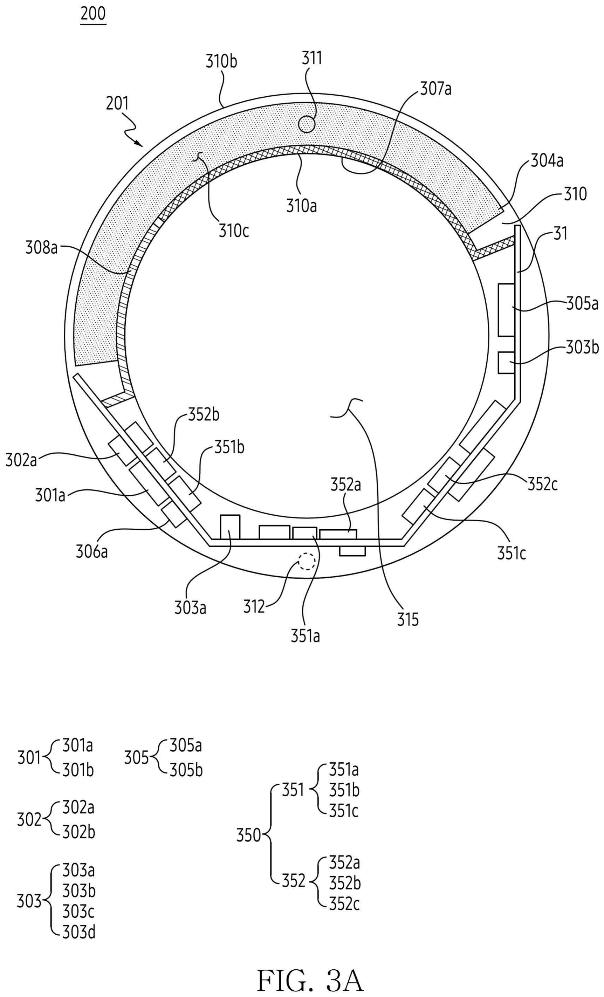

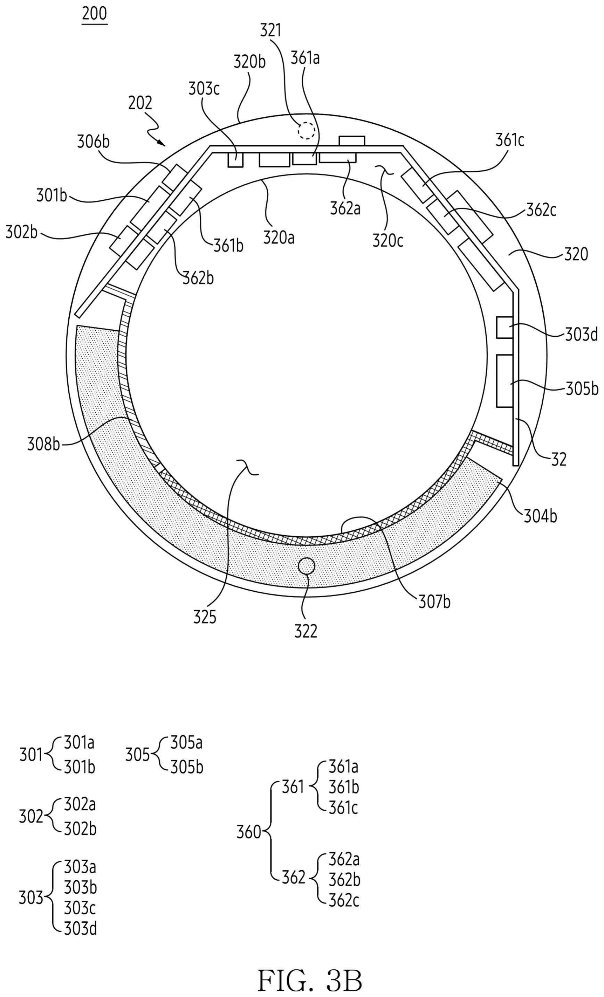

According to an embodiment, the wearable device 200 may include at least one electronic component (e.g., electronic components of FIG. 3A and/or electronic components of FIG. 3B) disposed in the housing 210. In order to provide various user experiences to the user, the wearable device 200 may include a structure in which two or more ring shaped devices including at least one electronic component are detachably coupled. As the two or more ring shaped devices are coupled with each other or the two or more ring shaped devices are separated, the structure in which the two or more ring shaped devices are detachably coupled may provide various user experiences to the user via an external electronic device (e.g., the electronic device 101). The structure in which the two or more ring shaped devices of the wearable device 200 are detachably coupled will be described below in FIG. 3A.

According to the above-described embodiment, the wearable device 200 may provide various user experiences to the user by being wearable on the portion 20 of the body of the user. The wearable device 200 may be configured to improve wearing comfort of the user and provide the user with the information related to the user, by including the housing 210 including the inner side 211 configured to face the portion 20 of the body of the user.

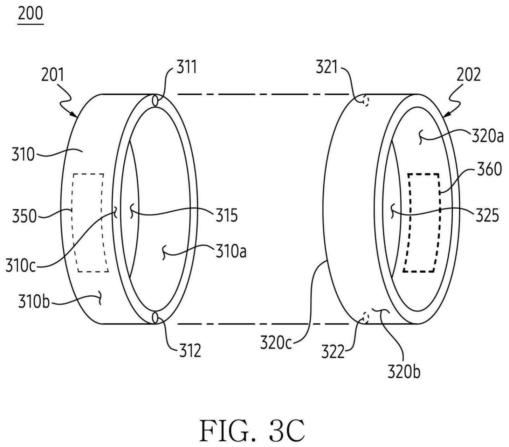

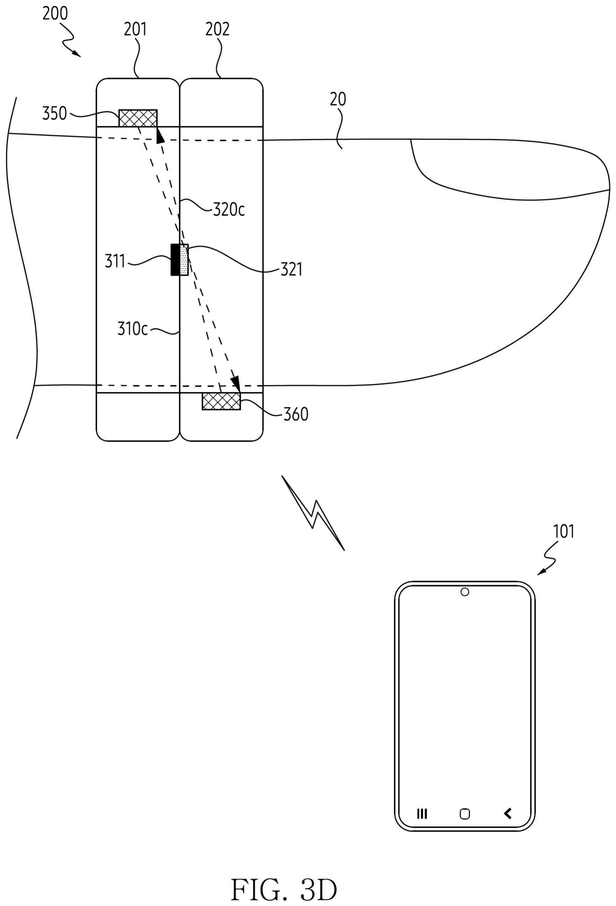

FIG. 3A illustrates a first ring of an exemplary wearable device. FIG. 3B illustrates a second ring of an exemplary wearable device. FIG. 3C illustrates a state in which a first ring and a second ring of an exemplary wearable device are separated. FIG. 3D illustrates a state in which a first ring and a second ring of an exemplary wearable device are coupled.

Referring to FIGS. 3A, 3B, 3C, and 3D, a wearable device 200 may include a first ring 201 and a second ring 202 detachably coupled with the first ring. According to an embodiment, the first ring 201 and the second ring 202 may each be referred to as a wearable device that may be worn by a user. The first ring 201 and the second ring 202 may respectively include electronic components in a first housing 310 and electronic components in a second housing 320 to perform functions of the first ring 201 and the second ring 202, respectively. For example, the wearable device 200 may include at least one processor 301, communication circuitry 302, a Hall sensor 303, and power management circuitry 305. The power management circuitry 305 may be implemented as at least a portion of a power management integrated circuit (PMIC).

The first ring 201 may include a first flexible printed circuit board 31 in the first housing 310. The first ring 201 may include a first processor 301a, first communication circuitry 302a, first Hall sensors 303a and 303b, first power management circuitry 305a, and a first memory 306a that are mounted on the first flexible printed circuit board 31. The first power management circuitry 305a may be configured to manage power supplied to the first ring 201. The first ring 201 may include a first battery 304a for supplying power to a plurality of electronic components mounted on the first flexible printed circuit board 31, a first charging interface 307a for receiving power from an external power source for charging the first battery 304a, and a first antenna 308a.

The at least one processor 301 may be configured to control at least a portion of electronic components in the wearable device 200. The at least one processor 301 may control the electronic components in the wearable device 200 via communication with an external electronic device (e.g., the electronic device 101 of FIG. 1) connected with the wearable device 200. For example, the first processor 301a in the first housing 310 may be configured to control at least a portion of electronic components of the first ring 201. A second processor 301b in the second housing 320 may be configured to control at least a portion of electronic components of the second ring 202.

The communication circuitry 302 may connect the external electronic device 101 and the wearable device 200. Via the communication circuitry 302, the at least one processor 301 may be configured to control at least a portion of the electronic components in the wearable device 200 or to control the external electronic device 101, based on a user input inputted to the external electronic device 101.

The Hall sensor 303 may be configured to sense a change in magnetic force around the Hall sensor 303. For example, the first Hall sensors 303a and 303b in the first housing 310 may sense a change in magnetic force by a first magnet 311 of the first housing 310. For example, second Hall sensors 303c and 303d in the second housing 320 may sense a change in magnetic force by a second magnet 321 of the second housing 320. The at least one processor 301 may be configured to identify whether the first magnet 311 and the second magnet 321 are coupled based on identifying the change in the magnetic force sensed from the Hall sensor 303. According to an embodiment, the at least one processor 301 may provide, via the communication circuitry 302, the external electronic device 101 with information indicating that the first housing 310 and the second housing 320 are coupled, based on identifying the coupling of the first magnet 311 and the second magnet 321. For example, the at least one processor 301 may identify the coupling of the first magnet 311 and the second magnet 321 and/or the coupling of the first ring 201 and the second ring 202 based on magnetic force between the first magnet 311 and the second magnet 321 sensed through the Hall sensor 303. The at least one processor 301 may be configured to connect the wearable device 200 to the external electronic device 101 via the communication circuitry 302 based on identifying the coupling of the first ring 201 and the second ring 202. Based on identifying the coupling of the first ring 201 and the second ring 202, the at least one processor 301 may display visual information indicating that the first ring 201 and the second ring 202 are coupled on a display (e.g., the display module 160 of FIG. 1) of the external electronic device 101 connected with the wearable device 200 via the communication circuitry 302 or may provide sound information indicating that the first ring 201 and the second ring 202 are coupled via an audio module (e.g., the audio module 170 of FIG. 1) of the external electronic device 101. However, it is not limited thereto.

According to an embodiment, the second ring 202 may be configured substantially the same as or similar to the first ring 201. For example, the second ring 202 may include the second processor 301b, second communication circuitry 302b, the second Hall sensors 303c and 303d, second power management circuitry 305b, and a second memory 306b that are mounted on a second flexible printed circuit board 32. The second power management circuitry 305b may be configured to manage power supplied to the second ring 202. The second ring 202 may include a second battery 304b for supplying power to a plurality of electronic components mounted on the second flexible printed circuit board 32, a second charging interface 307b for receiving power from an external power source for charging the second battery 304b, and a second antenna 308b.

Electronic components included in the first ring 201 and the second ring 202 of the wearable device 200 are not limited to the above-described configuration. For example, the first ring 201 and the second ring 202 may each include various sensors including a temperature sensor, a proximity sensor, a motion sensor, and a pressure sensor.

According to an embodiment, the first ring 201 may include the first housing 310 including the first magnet 311 and a first sensor 350 in the first housing 310. The second ring 202 may include the second housing 320 including the second magnet 321 and a second sensor 360 in the second housing 320.

For example, the first housing 310 may include a first side 310a facing a portion 20 of a body of the user when the user wears the wearable device 200, and a second side 310b opposite to the first side 310a. The first ring 201 may include a plurality of electronic components disposed between the first side 310a and the second side 310b. For example, the first sensor 350 may face the first side 310a of the first housing 310 to sense information related to the user via the portion 20 of the body of the user on which the wearable device 200 is worn. For example, the first magnet 311 may be at least partially exposed to the outside of the first housing 310. The first magnet 311 may be attached to the second magnet 321 to be detachably coupled with the second magnet 321 of the second ring 202. For example, the first magnet 311 may be configured to detachably couple the first housing 310 to the second housing 320 via the magnetic force between the first magnet 311 and the second magnet 321.

For example, the second housing 320 may be configured substantially the same as or similar to the first housing 210. For example, the second housing 320 may include a third side 320a facing a portion of the body of the user when the user wears the wearable device 200, and a fourth side 320b opposite to the third side 320a. The second ring 202 may include a plurality of electronic components disposed between the third side 320a and the fourth side 320b. For example, the second sensor 360 may face the third side 320b of the second housing 320 to sense information related to the user via the portion 20 of the body of the user on which the wearable device 200 is worn. For example, the second magnet 321 may be at least partially exposed to the outside of the second housing 320. The second magnet 321 may be attached to the first magnet 311 to be detachable from the first magnet 311 of the first ring 201. For example, the second magnet 321 may detachably couple the second housing 320 to the first housing 310 via the first magnet 311 by having a polarity opposite to a polarity of the first magnet 311.

According to an embodiment, the first sensor 350 may include a first light emitting portion 351 configured to emit light toward the portion 20 of the body of the user on which the wearable device 200 is worn, and a first light receiving portion 352 configured to receive at least a portion of the light emitted from the first light emitting portion 351 and reflected from the portion 20 of the body of the user. For example, the first light emitting portion 351 may include a plurality of light emitting portions 351a, 351b, and 351c. The plurality of light emitting portions 351a, 351b, and 351c may each face the first side 310a of the first housing 310 to emit light toward the portion 20 of the body of the user on which the wearable device 200 is worn. For example, the first light receiving portion 352 may include a plurality of light receiving portions 352a, 352b, and 352c. The plurality of light receiving portions 352a, 352b, and 352c may each face the first side 310a of the first housing 310 to each receive at least a portion of the light emitted from the first light emitting portion 351 and reflected by the portion 20 of the body of the user wearing the wearable device 200. For example, as at least a portion of the light emitted from the first light emitting portion 351 and reflected by the portion 20 of the body of the user is received by the first light receiving portion 352, the first sensor 350 may be configured to sense biometric information of the user.

According to an embodiment, the second sensor 360 may be configured substantially the same as or similar to the first sensor 350. For example, the second sensor 360 may include a second light emitting portion 361 configured to emit light toward the portion 20 of the body of the user on which the wearable device 200 is worn, and a second light receiving portion 362 configured to receive at least a portion of the light emitted from the second light emitting portion 361 and reflected from the portion 20 of the body of the user. For example, the second light emitting portion 361 may include a plurality of other light emitting portions 361a, 361b, and 361c facing the third side 320a of the second housing 320. The second light receiving portion 362 may include a plurality of other light receiving portions 362a, 362b, and 362c for receiving at least a portion of the light emitted from the second light emitting portion 361 and reflected by the portion 20 of the body of the user.

According to an embodiment, the first sensor 350 and the second sensor 360 may each include at least one of an optical sensor module and a heart rate measurement (HRM) sensor module using photoplethysmography (PPG), but are not limited thereto. The first light emitting portion 351 and the second light emitting portion 361 may be referred to as a light emitting diode (LED), and the first light receiving portion 352 and the second light receiving portion 362 may be referred to as a photo diode, but are not limited thereto.

According to an embodiment, in a case that only one ring among the first ring 201 and the second ring 202 is worn on the portion 20 of the body of the user, accuracy of information (e.g., biometric information) related to the user obtained through the first sensor 350 or the second sensor 360 may decrease according to a position of the first sensor 350 or the second sensor 360. The wearable device 200 may be required to have a structure for increasing the accuracy of the information related to the user.

According to an embodiment, via the second light receiving portion 362, the second sensor 360 may be configured to receive at least a portion of light being emitted from the first light emitting portion 351 and passing through the portion 20 of the body of the user via the second housing 320 coupled to the first housing 310. For example, the second housing 320 may be detachably coupled with the first housing 310 via the magnetic force between the first magnet 311 and the second magnet 321. As the second housing 320 is detachably coupled with the first housing 310, the second ring 202 may be coupled with the first ring 201. In a state in which the first ring 201 and the second ring 202 are coupled, the second sensor 360 in the second ring 202 may be disposed above the first ring 201.

For example, the first housing 310 may include a first hole 315 for passing the portion 20 of the body of the user. The second housing 320 may further include a second hole 325 configured to pass the portion 20 of the body of the user by being connected with the first hole 315 while the first ring 201 is coupled with the second ring 202. While the first housing 310 and the second housing 320 are coupled by the magnetic force between the first magnet 311 and the second magnet 321, at least a portion of the light emitted from the first light emitting portion 351 of the first sensor 350 may be received, across the first hole 315 and the second hole 325, by the second light receiving portion 362 of the second sensor 360. For example, while the first ring 201 and the second ring 202 are coupled, the wearable device 200 may be worn on the portion 20 of the body of the user, as the first hole 315 and the second hole 325 are penetrated by the portion 20 of the body of the user. At least a portion of the light emitted from the first light emitting portion 351 may be received by the second light receiving portion 362, by passing through the portion 20 of the body of the user penetrating the first hole 315 and the second hole 325. For example, the first sensor 350 may emit light toward the first side 310a of the first housing 310 using the first light emitting portion 351. At least a portion of the light emitted from the first light emitting portion 351 toward the first side 310a may be transmitted to the second light receiving portion 362 via the third side 320a of the second housing 320 by passing through the portion 20 of the body of the user. For example, as the second magnet 321 is attached to the first magnet 311, a position of the second sensor 360 relative to the first sensor 350 may be limited so that the second light receiving portion 362 receives at least a portion of the light from the first sensor 350. The second sensor 360 relative to the first sensor 350 may increase the accuracy of the information related to the user provided by the wearable device 200, by being positioned to be configured to receive at least a portion of the light being emitted from the first light emitting portion 351 of the first sensor 350 and passing through the portion 20 of the body of the user by the magnetic force between the first magnet 311 and the second magnet 321.

According to an embodiment, via the first light receiving portion 352, the first sensor 350 may be configured to receive at least a portion of light being emitted from the second light emitting portion 361 and passing through the portion 20 of the body of the user via the second housing 320 coupled to the first housing 310. For example, while the first housing 310 and the second housing 320 are coupled by the magnetic force between the first magnet 311 and the second magnet 321, at least a portion of the light emitted from the second light emitting portion 361 of the second sensor 360 may be received, across the second hole 325 and the first hole 315, by the first light receiving portion 352 of the first sensor 350. For example, while the first ring 201 and the second ring 202 are coupled, the wearable device 200 may be worn on the portion 20 of the body of the user as the first hole 315 and the second hole 325 are penetrated the portion 20 of the body of the user. At least a portion of the light emitted from the second light emitting portion 361 may be received by the first light receiving portion 352, by passing through the portion 20 of the body of the user penetrating the second hole 325 and the first hole 315. For example, the second sensor 360 may emit light toward the third side 320a of the second housing 320 using the second light emitting portion 361. At least a portion of the light emitted from the second light emitting portion 361 toward the third side 320a may be transmitted to the first light receiving portion 352 via the first side 310a of the first housing 310, by passing through the portion 20 of the body of the user. For example, as the second magnet 321 is attached to the first magnet 311, a position of the first sensor 350 relative to the second sensor 360 may be limited so that the first light receiving portion 352 receives at least a portion of the light from the second sensor 360. The first sensor 350 may increase the accuracy of the information related to the user provided by the wearable device 200, by being positioned to be configured to receive at least a portion of the light being emitted from the second light emitting portion 361 of the second sensor 360 and passing through the portion 20 of the body of the user by the magnetic force between the first magnet 311 and the second magnet 321.

According to an embodiment, the first housing 310 may include a first lateral side 310c on which the first magnet 311 is disposed. The second housing 320 may include a second lateral side 320c, on which the second magnet 321 is disposed, configured to be attached to the first lateral side 310c by the magnetic force between the first magnet 311 and the second magnet 321. For example, the first lateral side 310c may extend from the first side 210a of the first housing 210 to the second side 210b. For example, the first magnet 311 may be attached on the first lateral side 310c. For example, the first magnet 311 may be at least partially exposed to the outside of the first housing 310 via the first lateral side 310c. For example, the second lateral side 320c may extend from the third side 320a of the second housing 210 to the fourth side 320b. For example, the second magnet 321 may be attached on the second lateral side 320c. For example, the second lateral side 320c may be detachably attached on the first lateral side 310c by the magnetic force between the first magnet 311 and the second magnet 321. For example, the second lateral side 320c may connect the second hole 325 to the first hole 315, by being attached to the first lateral side 310c by the first magnet 311 and the second magnet 321. The second lateral side 320c may be configured such that the light emitted from the first light emitting portion 351 is received, across the first hole 315 and the second hole 325, by the second light receiving portion 362, by being attached to the first lateral side 310c. The first lateral side 310c may be configured such that the light emitted from the second light emitting portion 361 is received, across the first hole 315 and the second hole 325, by the first light receiving portion 352, by being attached to the second lateral side 320c.

According to an embodiment, the first housing 310 may include a third magnet 312 disposed on the first lateral side 310c and facing the first magnet 311. The second housing 320 may include a fourth magnet 322 disposed on the second lateral side 320c and facing the second magnet 321. The position of the second sensor 360 relative to the first sensor 350 may be configured to be fixed by the magnetic force between the first magnet 311 and the second magnet 321 and magnetic force between the third magnet 312 and the fourth magnet 322.

For example, the first magnet 311 may be attached to the second magnet 321 by attractive force with the second magnet 321. The third magnet 312 may be attached to the fourth magnet 322 by attractive force with the fourth magnet 322. The first magnet 311 may push the fourth magnet 322 by repulsive force. The second magnet 321 may push the third magnet 321 by repulsive force. Since the first magnet 311 is configured to be attached to the second magnet 321 and the third magnet 312 is configured to be attached to the fourth magnet 322, the wearable device 200 may fix a position of the second ring 201 relative to the first ring 201. The second ring 201 may be fastened to the first ring 201, so that at least a portion of the light emitted from the first light emitting portion 351 in the first sensor 350 is received by the second light receiving portion 362 in the second sensor 360 by passing through the portion 20 of the body of the user on which the wearable device 200 is worn. The first ring 201 may be fastened to the second ring 202, so that at least a portion of the light emitted from the second light emitting portion 361 in the second sensor 360 is received by the first light receiving portion 352 in the first sensor 350 by passing through the portion 20 of the body of the user on which the wearable device 200 is worn. The wearable device 200 may increase accuracy of information related to the user provided by the sensor modules 350 and 360 by including the magnets 311, 312, 321, and 322 configured to fix the position of the second sensor 360 relative to the first sensor 350.

According to an embodiment, the second sensor 360 may face the first sensor 350 while the first ring 201 is coupled with the second ring 202. For example, while the first ring 201 and the second ring 202 are coupled by the magnetic force between the first magnet 311 and the second magnet 321, the second sensor 360 may be positioned symmetrically relative to the first sensor 350. For example, while the first ring 201 is coupled with the second ring 202, the second sensor 360 may be disposed adjacent to a portion of the first side 310a that the first sensor 350 faces. The first sensor 350 may be disposed adjacent to a portion of the third side 320a that the second sensor 360 faces. The second sensor 360 may increase the accuracy of the information related to the user provided by the wearable device 200, by being configured to face the first sensor 350 while the first ring 201 is coupled with the second ring 202. For example, an angle between an axis connecting a center of the first hole 315 and the first sensor 350, and an axis connecting a center of the second hole 325 and the second sensor 360 may be positioned within a range of approximately 90 degrees to 180 degrees or less. However, it is not limited thereto.

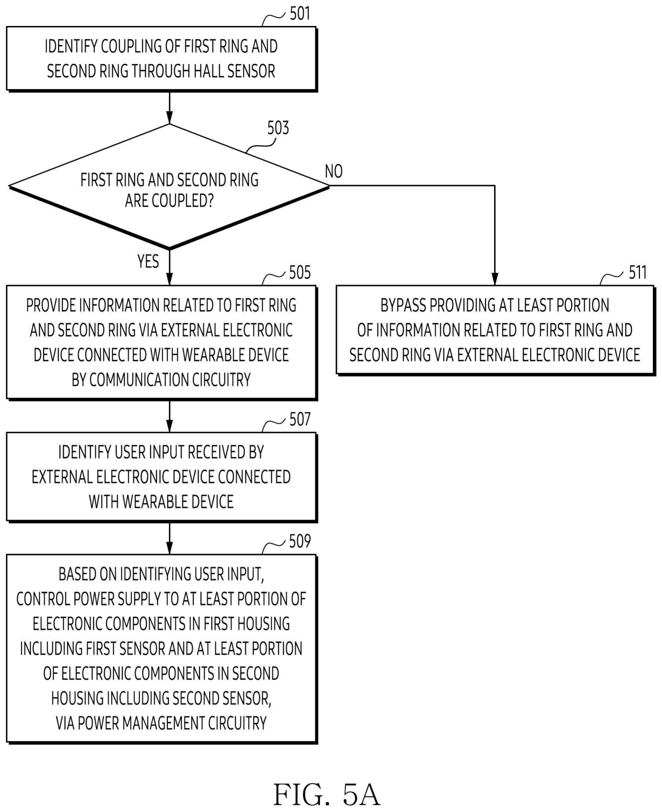

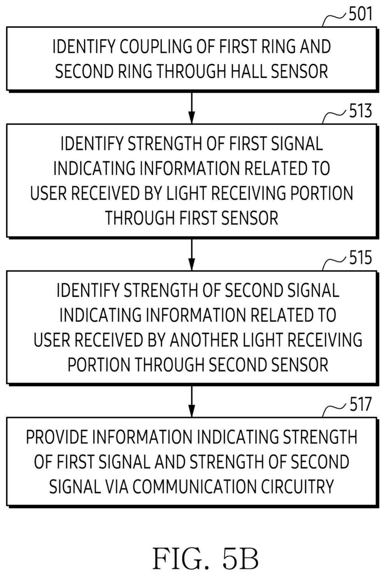

According to an embodiment, the wearable device 200 may provide information related to the electronic components of the first ring 201 and/or the electronic components of the second ring 202, via the external electronic device 101 connected with the wearable device 200 via the communication circuitry 302. Based on a user input received by the external electronic device 101, the at least one processor 301 may control at least a portion of the electronic components of the first ring 201 and/or at least a portion of the electronic components of the second ring 202. Based on a user input received by the external electronic device 101, the at least one processor 301 may be configured to bypass power supply to at least one of the first sensor 350 or the second sensor 360 via the power management circuitry 305. For example, based on a user input received by the external electronic device 101, the at least one processor 301 may be configured to bypass power supply to at least one of the first light emitting portion 351 and the second light emitting portion 361 and bypass power supply to at least one of the second light receiving portion 362 and the second light receiving portion 362, via the power management circuitry 305. The wearable device 200 may reduce unnecessary power consumption and provide various user experiences to the user, by being configured to control power supply to the electronic components in the wearable device 200 based on the coupling of the first ring 201 and the second ring 202. Controlling the electronic components in the wearable device 200 based on the user input will be described later with reference to FIGS. 5A and 5B.

According to the above-described embodiment, the wearable device 200 may provide various user experiences to the user by including the first ring 201 and the second ring 202 detachably coupled with the first ring 201. While the first ring 201 and the second ring 202 are coupled, the wearable device 200 may increase the accuracy of the information related to the user provided by the sensor modules 350 and 360, by including the magnets 311, 312, 321, and 322 configured to fix the position of the second sensor 360 of the second ring 202 relative to the first sensor 350 of the first ring 201.

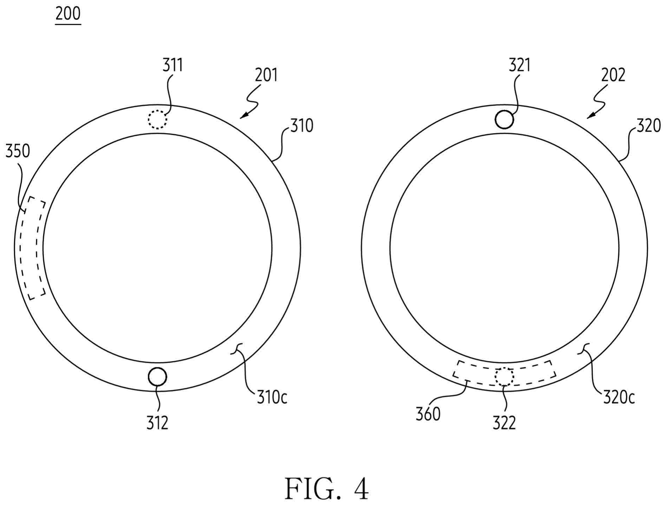

FIG. 4 illustrates an exemplary wearable device.

Referring to FIG. 4, a wearable device 200 may include a first ring 201 including a first housing 310 including a first magnet 311, and a first sensor 350 in the first housing 310. The wearable device 200 may include a second ring 202 including a second housing including a second magnet 321 and a second sensor 360 in the second housing 320, and being detachably coupled with the first ring 201 by magnetic force between the first magnet 311 and the second magnet 321. The first sensor 350 may include a first light emitting portion (e.g., the first light emitting portion 351 of FIG. 3A) configured to emit light toward a portion of a body (e.g., the portion 20 of the body of FIG. 2) of a user on which the wearable device 200 is worn, and a first light receiving portion (e.g., the first light receiving portion 352 of FIG. 3A). The second sensor 360 may include a second light emitting portion (e.g., the second light emitting portion 361 of FIG. 3B) and a second light receiving portion (e.g., the second light receiving portion 362 of FIG. 3B) configured to receive, via the second housing 320 coupled to the first housing 310, at least a portion of the light being emitted from the first light emitting portion 351 and passing through the portion 20 of the body of the user.

Hereinafter, overlapping descriptions of the configurations described in FIGS. 3A to 3D will be omitted.

Referring to FIG. 4, unlike FIGS. 3A to 3D, while the first ring 201 and the second ring 202 are coupled by the first magnet 311 and the second magnet 321, the second sensor 360 may not face the first sensor 350. For example, at least a portion of the light emitted from the first light emitting portion 351 of the first sensor 350 may be reflected by the portion 20 of the body of the user and then received by the second light receiving portion 362 of the second sensor 360. The second sensor 360 may be configured to sense biometric information of the user via at least a portion of the light emitted from the first light emitting portion 351. For example, at least a portion of light emitted from the second light emitting portion 361 of the second sensor 360 may be reflected by the portion 20 of the body of the user and then received by the first light receiving portion 352 of the first sensor 350. The first sensor 350 may be configured to sense biometric information of the user via at least a portion of the light emitted from the second light emitting portion 361. For example, the first housing 310 may include a first hole 315, and the second housing 320 may include a second hole 325 connected with the first hole 315 while the first ring 201 and the second ring 202 are coupled. An angle between an axis connecting a center of the first hole 315 and the first sensor 350 and an axis connecting a center of the second hole 325 and the second sensor 360 may be within a range of approximately 0 degrees to 90 degrees or less. However, it is not limited thereto.

According to the above-described embodiment, while the first ring 201 and the second ring 202 are coupled, the wearable device 200 may increase accuracy of information related to the user provided by the sensor modules 350 and 360, by being configured to fix a position of the second sensor 360 of the second ring 202 relative to the first sensor 350 of the first ring 201.

FIGS. 5A and 5B are flow charts indicating an operation of a processor of an exemplary wearable device.

In the following embodiment, each of operations may be sequentially performed, but is not necessarily performed sequentially. For example, an order of each of the operation may be changed, and at least two operations may be performed in parallel.

The operations of FIGS. 5A and 5B may be performed by the at least one processor 301 of FIGS. 3A and 3B.

Referring to FIGS. 5A and 5B, in operation 501, the at least one processor 301 may be configured to identify a coupling of a first ring (e.g., the first ring 201 of FIG. 3A) and a second ring (e.g., the second ring 202 of FIG. 3B) by a Hall sensor (e.g., the Hall sensor 303 of FIG. 3A). For example, the at least one processor 301 may identify the coupling of the first ring 201 and the second ring 202 via a change in magnetic force between a first magnet (e.g., the first magnet 311 of FIG. 3A) and a second magnet (e.g., the second magnet 321 of FIG. 3B) sensed through the Hall sensor 303.

Referring to FIG. 5A, in operation 503, the at least one processor 301 may be configured to identify whether the first ring 201 and the second ring 202 are coupled. For example, the at least one processor 301 may identify the coupling of the first ring 201 and the second ring 202 based on identifying attractive force between the first magnet 311 and the second magnet 321 sensed through the Hall sensor 303. For example, the at least one processor 301 may identify that the first ring 201 and the second ring 202 are separated, based on identifying that the magnetic force is not sensed through the Hall sensor 303.

In operation 505, the at least one processor 301 may be configured to provide information related to the first ring 201 and the second ring 202 via an external electronic device (e.g., the electronic device 101 of FIG. 1) connected with the wearable device 200 by communication circuitry (e.g., the communication circuitry 302 of FIG. 3A), based on identifying that the first ring 201 and the second ring 202 are coupled. For example, via first communication circuitry (e.g., the first communication circuitry 302a of FIG. 3A), a first processor (e.g., the first processor 301a of FIG. 3A) of the first ring 201 may provide information related to the first ring 201 or information related to the second ring 202 obtained via a first antenna (e.g., the first antenna 308a of FIG. 3A), via the external electronic device 101. For example, via second communication circuitry (e.g., the second communication circuitry 302b of FIG. 3B), a second processor (e.g., the second processor 301b of FIG. 3B) of the second ring 202 may provide information related to the first ring 201 or information related to the second ring 202 obtained via a second antenna (e.g., the second antenna 308b of FIG. 3B), via the external electronic device 101.

In operation 507, the at least one processor 301 may be configured to identify a user input received by the external electronic device 101 connected with the wearable device 200 via the communication circuitry 302. For example, via the communication circuitry 302, the at least one processor 301 may be configured to identify the user input received by the external electronic device 101 via a processor (e.g., the processor 120 of FIG. 1) of the external electronic device 101 and communication circuitry (e.g., the communication circuitry 190 of FIG. 1) of the external electronic device 101.

In operation 509, based on identifying a user input, the at least one processor 301 may be configured to control power supply to at least a portion of electronic components in a first housing (e.g., the first housing 310 of FIG. 3A) including a first sensor (e.g., the first sensor 350 of FIG. 3A) and at least a portion of electronic components in a second housing (e.g., the second housing 320 of FIG. 3B) including a second sensor (e.g., the second sensor 360 of FIG. 3B) via power management circuitry (e.g., the power management circuitry 305 of FIG. 3B). For example, a user input inputted to the external electronic device 101 may be an input for bypassing power supply to at least a portion of electronic components of the first ring 201 and/or at least a portion of electronic components of the second ring 202. For example, based on identifying a user input, the first processor 301a may be configured to control first power management circuitry (e.g., the first power management circuitry 305a of FIG. 3A) to bypass power supply to at least one of a first light emitting portion (e.g., the first light emitting portion 351 of FIG. 3A) and a first light receiving portion (e.g., the first light receiving portion 352 of FIG. 3A) of the first sensor 350. For example, based on identifying a user input, the second processor 301b may be configured to control second power management circuitry (e.g., the second power management circuitry 305b of FIG. 3B) to bypass power supply to at least one of a second light emitting portion (e.g., the second light emitting portion 361 of FIG. 3B) and a second light receiving portion (e.g., the second light receiving portion 362 of FIG. 3B) of the second sensor 360. For example, the at least one processor 301 may turn off power of the first ring 201 or turn off power of the second ring 202 via the power management circuitry 305. However, it is not limited to thereto, and the at least one processor 301 may be configured to bypass power supply to at least a portion of the electronic components illustrated in FIGS. 3A and 3B, based on a user input received from the external electronic device 101.

In operation 511, the at least one processor 301 may be configured to bypass providing at least a portion of information related to the first ring 201 and the second ring 202 via the external electronic device 101, based on identifying that the first ring 201 and the second ring 202 are separated. For example, the at least one processor 301 may be configured to bypass providing at least a portion of the information related to the first ring 201 and the second ring 202 based on the magnetic force sensed by the Hall sensor 303 not being identified.

Referring to FIG. 5B, in operation 513, the at least one processor 301 may be configured to identify a strength s1 of a first signal indicating information related to the user received by a light receiving portion (e.g., the first light receiving portion 352 of FIG. 3A) through the first sensor 350, based on identifying the coupling of the first ring 201 and the second ring 202. For example, the at least one processor 301 may identify a strength of a signal emitted from the first light emitting portion 351 and reflected by a portion of a body of the user (e.g., the portion 20 of the body of FIG. 2), and transmitted to the first light receiving portion 352. For example, the at least one processor 301 may identify a strength of a signal being emitted from the second light emitting portion 361 and passing through the portion 20 of the body of the user, and being transmitted to the first light receiving portion 352.

In operation 515, the at least one processor 301 may be configured to identify a strength s2 of a second signal indicating information related to the user received by another light receiving portion (e.g., the second light receiving portion 362 of FIG. 3B ) through the second sensor 360, based on identifying the coupling of the first ring 201 and the second ring 202. For example, the at least one processor 301 may identify a strength of a signal emitted from the second light emitting portion 361 and reflected by the portion 20 of the body of the user, and transmitted to the second light receiving portion 362. For example, the at least one processor 301 may identify a strength of a signal being emitted from the first light emitting portion 351 and passing through the portion 20 of the body of the user, and being transmitted to the second light receiving portion 362.

In operation 517, the at least one processor 301 may provide information indicating the strength s1 of the first signal and the strength s2 of the second signal via the communication circuitry 302, by using the external electronic device 101. For example, the at least one processor 301 may provide various user experiences related to the first sensor 350 or the second sensor 360 to the user, by providing the information related to the strength s1 of the first signal and the strength s2 of the second signal via the communication circuitry 302 and the processor 120 of the external electronic device 101.

According to the above-described embodiment, the at least one processor 301 of the wearable device 200 may provide various user experiences to the user, by being configured to control the electronic components of the first ring 201 and the second ring 202 via the external electronic device 101.

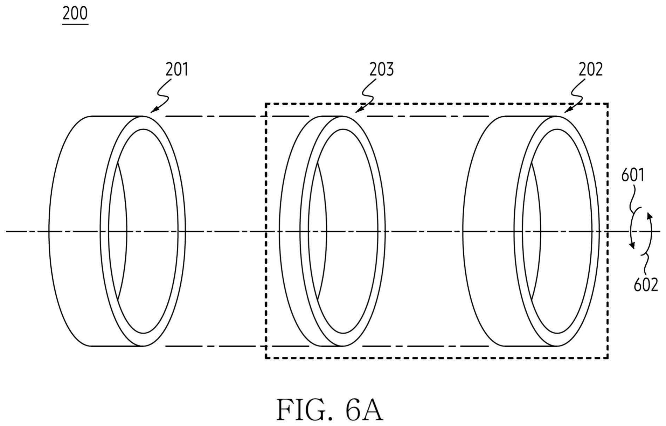

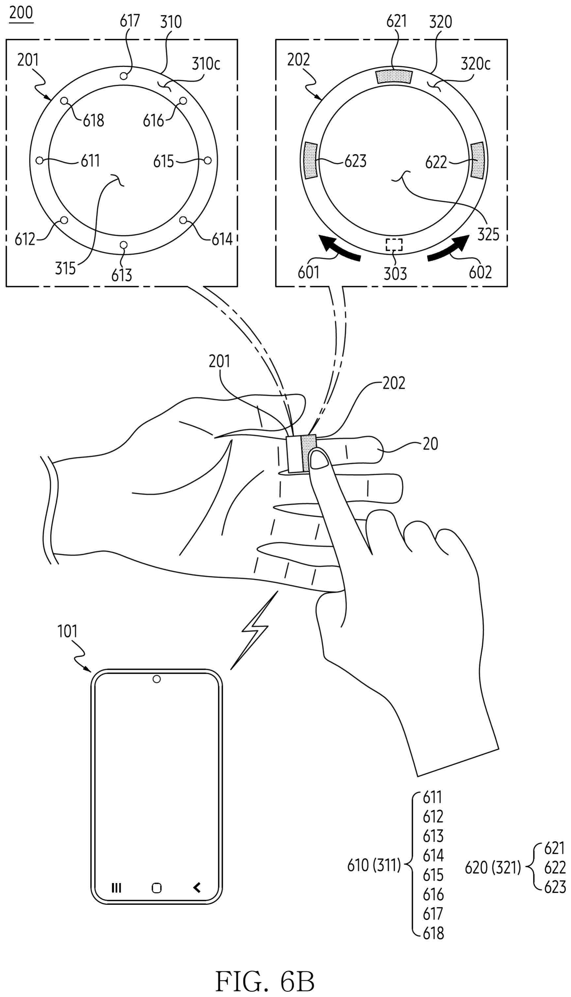

FIGS. 6A and 6B illustrate an exemplary wearable device.

Referring to FIGS. 6A and 6B, a wearable device 200 may include a first ring 201 including a first housing 310 including a first magnet 311 and a first sensor 350 in the first housing 310. The wearable device 200 may include a second ring 202 including a second housing including a second magnet 321 and a second sensor 360 in the second housing 320, and being detachably coupled with the first ring 201 by magnetic force between the first magnet 311 and the second magnet 321. The first sensor 350 may include a first light emitting portion (e.g., the first light emitting portion 351 of FIG. 3A) configured to emit light toward a portion of a body (e.g., the portion 20 of the body of FIG. 2) of a user on which the wearable device 200 is worn, and a first light receiving portion (e.g., the first light receiving portion 352 of FIG. 3A). The second sensor 360 may include a second light emitting portion (e.g., the second light emitting portion 361 of FIG. 3B) and a second light receiving portion (e.g., the second light receiving portion 362 of FIG. 3B) configured to receive, via the second housing 320 coupled to the first housing 310, at least a portion of the light being emitted from the first light emitting portion 351 and passing through the portion 20 of the body of the user. According to an embodiment, the first housing 310 may further include a first hole 315 for passing the portion 20 of the body of the user. The second housing 320 may further include a second hole 325 configured to pass the portion 20 of the body of the user by being connected with the first hole 315 while the first ring 201 is coupled with the second ring 202.

Referring to FIG. 6A, the wearable device 200 may include a third ring 203 disposed between the first ring 201 and the second ring 202 while the first ring 201 and the second ring 202 are coupled. The first ring 201 and the second ring 202 may be each rotatable relative to the third ring 203. For example, the third ring 203 may include a material having a magnetic property. For example, the first ring 201 may be attached to a lateral side of the third ring 203 by magnetic force between the first magnet 311 and the third ring 203. For example, the second ring 202 may be attached to another side opposite to the side of the third ring 203 by magnetic force between the second magnet 321 and the third ring 203. For example, the third ring 203 may be referred to as a metal ring, but is not limited thereto.

For example, the first ring 201 and the second ring 202 may be rotatable in a state of being attached to the third ring 203. The first ring 201 and/or the second ring 202 may be each rotatable relative to the third ring 203 in a first rotation direction 601 and a second rotation direction 602 opposite to the first rotation direction. For example, a Hall sensor (e.g., the Hall sensor 303 of FIG. 3A) in the wearable device 200 may sense a change in the magnetic force between the first magnet 311 and the third ring 203 and/or a change in the magnetic force between the second magnet 321 and the third ring 203. At least one processor (e.g., the at least one processor 301 of FIG. 3A) in the wearable device 200 may be configured to identify a rotation of the first ring 201 and/or a rotation of the second ring 202 relative to the third ring 203, based on the change in the magnetic force sensed from the Hall sensor 303. Based on identifying the rotation of the first ring 201 and/or the rotation of the second ring 202 relative to the third ring 203, the at least one processor 301 may be configured to control an external electronic device 101 connected with the wearable device 200. For example, based on identifying the rotation of the first ring 201 and/or the rotation of the second ring 202, the at least one processor 301 may control the external electronic device 101 to adjust a volume of a speaker (e.g., the audio module 170 of FIG. 1) in the external electronic device 101 via a processor (e.g., the processor 120 of FIG. 1) in the external electronic device 101. For example, based on identifying the rotation of the first ring 201 and/or the rotation of the second ring 202, the at least one processor 301 may control the external electronic device 101 to change visual information displayed by a display (e.g., the display module 160 of FIG. 1) of the external electronic device 101 via the processor 120 in the external electronic device 101. For example, based on identifying the rotation of the first ring 201 and/or the rotation of the second ring 202, the at least one processor 301 may control the external electronic device 101 to cause zoom in or zoom out of a camera (e.g., the camera module 180 of FIG. 1) of the external electronic device 101 via the processor 120 in the external electronic device 101. However, it is not limited thereto.

Although the third ring 203 is described as being disposed between the first ring 201 and the second ring 202 and rotatably coupling the first ring 201 and the second ring 202 based on the third ring 203, but is not limited thereto. The third ring 203 may include a plurality of electronic components as described in FIGS. 3A and 3B. The third ring 203 may provide information related to whether it is coupled with the first ring 201 and/or the second ring 202 via the external electronic device 101 connected with the third ring 203, by including the at least one processor 301. However, it is not limited thereto, and the wearable device 200 may be configured to generate an event for executing a function of the external electronic device 101 in the external electronic device 101 as three or more ring shaped devices are detachably coupled with each other, or to allow each of the plurality of ring shaped devices to share a function of a plurality of electronic components included in the plurality of ring shaped devices.

Referring to FIG. 6B, the first magnet 311 may include a first set of magnets 610 spaced apart from each other at a designated interval along the first hole 315. The second ring 202 may be configured to be rotatable relative to the first ring 201 via magnetic force between the first set of magnets 610 and the second magnet 321 while coupled with the first ring 201.

For example, the first set of magnets 610 may include first magnets 611, 612, 613, 614, 615, 616, 617, and 618 spaced apart at the designated interval. As the second ring 202 rotates relative to the first ring 201 in a first rotation direction 601 and a second rotation direction 602 opposite to the first rotation direction 601, the second magnet 321 may be positioned on the first magnets 611, 612, 613, 614, 615, 616, 617, and 618, respectively. Since each of the first magnets 611, 612, 613, 614, 615, 616, 617, and 618 and the second magnet 321 have different polarities from each other, the second ring 202 may maintain a coupling with the first ring 201 via magnetic force between each of the first magnets 611, 612, 613, 614, 615, 616, 617, and 618 and the second magnet 321 while rotating relative to the first ring 201. For example, the second magnet 321 may include a second set of magnets 620 spaced apart at a designated interval. Via magnetic force with the first set of magnets 610, the second set of magnets 620 may reduce separation of the second ring 202 from the first ring 201 while the second ring 202 rotates relative to the first ring 201 in the first rotation direction 601 and in the second rotation direction 602 opposite to the first rotation direction.

For example, the second magnet 321 may include the second set of magnets 620 having different polarities from the first set of magnets 610 and being spaced apart from each other to be respectively coupled with at least a portion of the first set of magnets 610. For example, the second set of magnets 620 may include second magnets 621, 622, and 623 spaced apart at a designated interval on a second lateral side 320c. Each of the second magnets 621, 622, and 623 may attach the second lateral side 320c to a first lateral side 310c via magnetic force with at least a portion of the first magnets 611, 612, 613, 614, 615, 616, 617, and 618, by being disposed at a position corresponding to the at least a portion of the first magnets 611, 612, 613, 614, 615, 616, 617, and 618.

According to an embodiment, the Hall sensor 303 may be configured to sense a change in the magnetic force between each of the first set of magnets 610 and the second magnet 321. For example, the Hall sensor 303 may be disposed in the second ring 202. The Hall sensor 303 may be attached to the second lateral side 320c on which the second magnet 321 of the second ring 202 is disposed. The second lateral side 320c may include a metal so as to be attached to the first lateral side 310c via the first set of magnets 610 on the first lateral side 310c. However, it is not limited thereto. Unlike the illustration, the Hall sensor 303 may be disposed in the first ring 201. The Hall sensor 303 may be attached to the first lateral side 310c on which the first set of magnets 610 of the first ring 201 are disposed. The first lateral side 310c may include a metal so as to be attached to the second lateral side 320c via the second magnet 321 on the second lateral side 320c. The at least one processor 301 of the wearable device 200 may be configured to identify a rotation and/or a rotation direction (e.g., the first rotation direction 601 and the second rotation direction 602) of the second ring 202 relative to the first ring 201 via a change in the magnetic force between the first set of magnets 610 and the second magnet 321 sensed through the Hall sensor 303. Based on identifying the rotation and/or the rotation direction of the second ring 202 relative to the first ring 201, the at least one processor 301 may be configured to control the external electronic device 101 via the processor 120 in the external electronic device 101. For example, based on identifying the rotation of the second ring 202 relative to the first ring 201, the at least one processor 301 may control the external electronic device 101 to adjust a volume of the speaker in the external electronic device 101, change visual information displayed by the display of the external electronic device 101, or cause zoom in or zoom out of the camera of the external electronic device 101 via the processor 120 of the external electronic device 101. However, it is not limited thereto.

According to the above-described embodiment, the wearable device 200 may be configured to control the external electronic device 101 connected with the wearable device 200 via the rotation of the second ring 202 relative to the first ring 201, by including the second ring 202 configured to be rotatably coupled to the first ring 201.

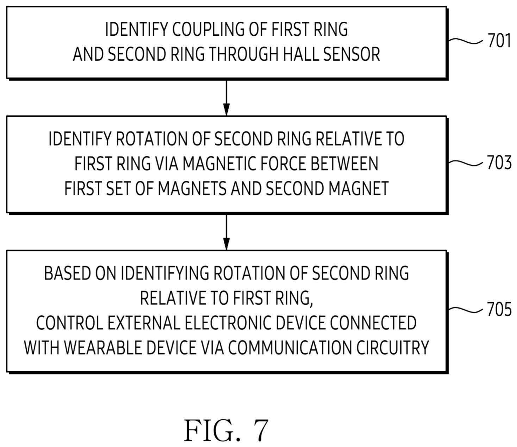

FIG. 7 is a flow chart indicating an operation of a processor of an exemplary wearable device.

In the following embodiment, each of operations may be sequentially performed, but is not necessarily performed sequentially. For example, an order of each of the operation may be changed, and at least two operations may be performed in parallel.

The operation of FIG. 7 may be performed by the at least one processor 301 of FIG. 3A. Operation 701 may be an operation corresponding to the operation 501 of FIGS. 5A and 5B.

In operation 703, based on identifying a coupling of the first ring (e.g., the first ring 201 of FIG. 3A) and the second ring (e.g., the second ring 202 of FIG. 3B), the at least one processor 301 may be configured to identify a rotation of the second ring 202 relative to the first ring 201, via the magnetic force between a first set of magnets (e.g., the first set of magnets 610 of FIG. 6B) and a second magnet (e.g., the second magnet 321 of FIG. 3B). For example, the at least one processor 301 may be configured to identify the rotation of the second ring 202 relative to the first ring 201 via a change in magnetic force sensed through a Hall sensor (e.g., the Hall sensor 303 of FIG. 3A). For example, the Hall sensor 303 may include a plurality of Hall sensors (e.g., the first Hall sensors 303a and 303b of FIG. 3A, and the second Hall sensors 303c and 303d of FIG. 3B) included in the first ring 201 and/or the second ring 202. Since the first ring 201 or the second ring 202 each includes a plurality of Hall sensors, the at least one processor 301 may be configured to obtain 2-axis data through the plurality of Hall sensors. The at least one processor 301 may be configured to identify the rotation of the second ring 202 relative to the first ring 201 and/or a rotation of the first ring 201 relative to the second ring 202 via at least a portion of the data obtained by the plurality of Hall sensors. For example, although not illustrated in FIGS. 3A and 3B, a wearable device 200 may further include a magnetic sensor. The at least one processor 301 may be configured to obtain 3-axis data through the magnetic sensor. The at least one processor 301 may be configured to identify the rotation of the second ring 202 relative to the first ring 201 and/or the rotation of the first ring 201 relative to the second ring 202 via at least a portion of the data obtained through the magnetic sensor.

In operation 705, based on identifying the rotation of the second ring 202 relative to the first ring 201, the at least one processor 301 may be configured to control the external electronic device (e.g., the electronic device 101 of FIG. 1) via communication circuitry (e.g., the communication circuitry 302 of FIG. 3A). For example, based on identifying the rotation of the second ring 202 relative to the first ring 201, the at least one processor 301 may control the external electronic device 101 to adjust a volume of the external electronic device 101. However, it is not limited thereto.

According to the above-described embodiment, the at least one processor 301 of the wearable device 200 may provide various user experiences to a user, by being configured to control the external electronic device 101 based on identifying the rotation of the second ring 202 relative to the first ring 201.

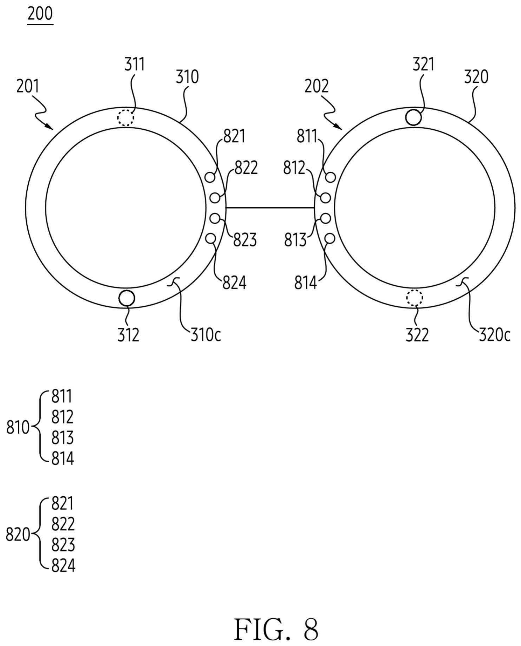

FIG. 8 illustrates an exemplary wearable device.

Referring to FIG. 8, a wearable device 200 may include a first ring 201 including a first housing 310 including a first magnet 311, and a first sensor 350 in the first housing 310. The wearable device 200 may include a second ring 202 including a second housing including a second magnet 321 and a second sensor 360 in the second housing 320, and being detachably coupled with the first ring 201 by magnetic force between the first magnet 311 and the second magnet 321. The first sensor 350 may include a first light emitting portion (e.g., the first light emitting portion 351 of FIG. 3A) configured to emit light toward a portion of a body (e.g., the portion 20 of the body of FIG. 2) of a user on which the wearable device 200 is worn, and a first light receiving portion (e.g., the first light receiving portion 352 of FIG. 3A). The second sensor 360 may include a second light emitting portion (e.g., the second light emitting portion 361 of FIG. 3B) and a second light receiving portion (e.g., the second light receiving portion 362 of FIG. 3B) configured to receive, via the second housing 320 coupled to the first housing 310, at least a portion of the light being emitted from the first light emitting portion 351 and passing through the portion 20 of the body of the user.

According to an embodiment, the second housing 320 may include at least one conductive pin 810. The first housing 310 may include at least one connector hole 820 electrically connected with the at least one conductive pin 810 while the first ring 201 is coupled with the second ring 202. For example, the first magnet 311 may be disposed on a first lateral side 310c of the first housing 310. The second magnet 321 may be disposed on a second lateral side 320c of the second housing 320. The at least one conductive pin 810 may protrude from the second lateral side 320c on which the second magnet 321 is disposed. The at least one connector hole 820 may be formed on the first lateral side 310c on which the first magnet 311 is disposed. For example, the at least one conductive pin 810 may include a plurality of conductive pins 811, 812, 813, and 814. The at least one connector hole 820 may include a plurality of connector holes 821, 822, 823, and 824. While the first ring 201 is coupled to the second ring 202, the plurality of conductive pins 811, 812, 813, and 814 may electrically connect the first ring 201 and the second ring 202 by being inserted into the plurality of connector holes 821, 822, 823, and 824.

According to an embodiment, the first ring 201 may include a first memory (e.g., the first memory 306a of FIG. 3A) in the first housing 310. The second ring 202 may include a second memory (e.g., the second memory 306b of FIG. 3B) in the second housing 320. The first ring 201 may be configured to transmit data from the first memory 306a to the second memory 306b or to receive data from the second memory 306b to the first memory 306a, via the at least one conductive pin 810 connected with the at least one connector hole 820. For example, based on a user input received from an external electronic device (e.g., the electronic device 101 of FIG. 1), a first processor (e.g., the first processor 301a of FIG. 3A) of the first ring 201 may be configured to transmit at least a portion of data stored in the first memory 306a to the second memory 306b. For example, based on a user input received from the external electronic device 101, a second processor (e.g., the second processor 301b of FIG. 3B) in the second ring 202 may be configured to transmit at least a portion of data stored in the second memory 306b to the first memory 306a.

According to an embodiment, the first ring 201 may include a first battery (e.g., the first battery 304a of FIG. 3A) for supplying power. The second ring 202 may include a second battery 304b for supplying power to the second ring 202. The first ring 201 may be configured to supply power from the first battery 304a to the second battery 304b or to receive power from the second battery 304b to the first battery 304a, via the at least one conductive pin 810 connected with the at least one connector hole 820. For example, based on a user input received from the external electronic device 101, the first processor 301a of the first ring 201 may be configured to supply power from the first battery 304a to the second battery 304b via first power management circuitry 305a. For example, based on a user input received from the external electronic device 101, the second processor 301b of the second ring 202 may be configured to supply power from the second battery 304b to the first battery 304a via second power management circuitry 305b.

For example, unlike the illustration in FIGS. 3A and 3B, the second ring 202 may have a configuration substantially different from that of the first ring 201. The second ring 202 may not include, for example, at least one processor 301. A size and/or a capacity of the second battery 304b disposed inside the second housing 320 of the second ring 202 may be greater than a size and/or a capacity of the first battery 304a in the first ring 201. The second ring 202 may be configured to charge the first ring 201 via the second battery 304b having the capacity larger than that of the first battery 304a, by being connected with the first ring 201. For example, a size and/or a capacity of the second memory 306b disposed inside the second housing 320 of the second ring 202 may be greater than a size and/or a capacity of the first memory 306a in the first ring 201. The second ring 202 may store data from the first ring 201 via the second memory 306b having the capacity larger than that of the first memory 306a by being connected with the first ring 201. However, it is not limited thereto. The wearable device 200 may share a function of electronic components disposed inside the first ring 201 and/or the second ring 202 and provide various user experiences to the user of the wearable device 200, by including the first ring 201 and the second ring 202 connected with each other and respectively including different configurations and/or electronic components.

According to the above-described embodiment, the wearable device 200 may connect the electronic components in the first ring 201 and the second ring 202 and provide various user experiences to the user, by including the at least one conductive pin 810 and the at least one connector hole 820 configured to be electrically connected with the at least one conductive pin 810.

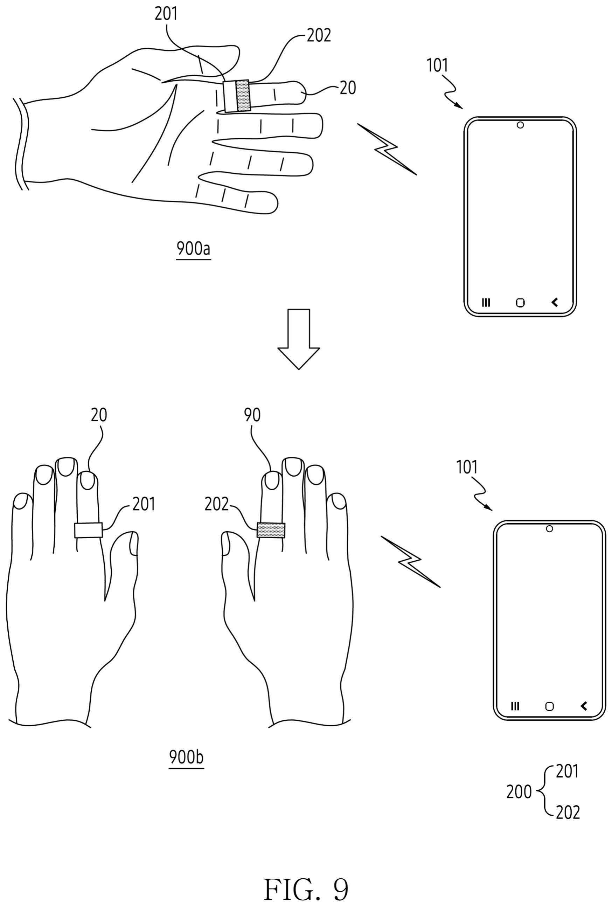

FIG. 9 illustrates an exemplary wearable device worn by a user.

Referring to FIG. 9, a wearable device 200 may include a first ring 201 and a second ring 202 detachably coupled with the first ring 201. The first ring 201 and the second ring 202 may include a first magnet (e.g., the first magnet 311 of FIG. 3A) and a second magnet (e.g., the second magnet 321 of FIG. 3B), respectively.

In a state 900a, the first ring 201 and the second ring 202 may be coupled with each other via the first magnet 311 and the second magnet 321, respectively. The first ring 201 and the second ring 202 may be worn on a portion 20 of a body of a user. By being coupled with each other, the first ring 201 and the second ring 202 may transmit information related to the first ring 201 and/or the second ring 202 to an external electronic device 101 connected with the wearable device 200 or receive data from the external electronic device 101 to the wearable device 200 based on a user input to the external electronic device 101.

In a state 900b, the first ring 201 and the second ring 202 may be separated from each other. For example, while changing from the state 900a to the state 900b, the first ring 201 may be worn on the portion 20 of the body of the user, and the second ring 202 may be worn on another portion 90 of the body of the user. In a case that the state 900a is changed to the state 900b, a connection between the first ring 201 and the second ring 202 may be maintained. For example, while changing from the state 900a to the state 900b, a first processor (e.g., the first processor 301a of FIG. 3A) in the first ring 201 may be configured to maintain receiving information related to the second ring 202 from the second ring 202. In the state 900b, the first processor 301a may provide the information related to the second ring 202 received from the second ring 202 via the external electronic device 101 connected with the first ring 201. For example, while changing from the state 900a to the state 900b, a second processor (e.g., the second processor 301b of FIG. 3B) in the second ring 202 may be configured to maintain receiving information related to the first ring 201 from the first ring 201. The second processor 301b may provide information related to the first ring 201 received from the first ring 201 via the external electronic device 101 connected to the second ring 202. However, it is not limited thereto, and the first ring 201 and the second ring 202 separated from each other while changing from the state 900a to the state 900b may each perform independent functions. The first ring 201 and/or the second ring 202 may be configured to provide information related to the wearable device 200 via the external electronic device 101, by being connected to communicate with each other in the state 900b.

According to the above- mentioned embodiment, a wearable device (e.g., the wearable device 200 of FIG. 2) may comprise a first ring (e.g., the first ring 201 of FIG. 3A) including a first housing (e.g., the first housing 310 of FIG. 3A) including a first magnet (e.g., the first magnet 311 of FIG. 3A), and a first sensor (e.g., the first sensor 350 of FIG. 3A) in the first housing. The wearable device may comprise a second ring (e.g., the second ring 202 of FIG. 3B) including a second housing (e.g., the second housing 320 of FIG. 3A) including a second magnet (e.g., the second magnet 321 of FIG. 3B), and a second sensor (e.g., the second sensor 360 of FIG. 3B) in the second housing, the second ring being detachably coupled with the first ring by magnetic force between the first magnet and the second magnet. The first sensor may include a light emitting portion (e.g., the first light emitting portion 351 of FIG. 3A) configured to emit light toward a portion of a body of a user on which the wearable device is worn. The second sensor may include a light receiving portion (e.g., the second light receiving portion 362 of FIG. 3B) configured to receive, via the second housing coupled to the first housing, at least a portion of the light being emitted from the light emitting portion and passing through the portion of the body of the user. According to the above-mentioned embodiment, the wearable device may provide accuracy of information related to the user provided from the first sensor and the second sensor, by providing a structure in which the first ring and the second ring may be coupled such that a position of the second sensor relative to the first sensor is fixed. The above-mentioned embodiment may have various effects including the above-mentioned effect.

According to an embodiment, the first housing may further include a first lateral side (e.g., the first lateral side 310c of FIG. 3A) on which the first magnet is disposed. The second housing may further include a second lateral side (e.g., the second lateral side 320c of FIG. 3B) on which the second magnet is disposed, the second lateral side being configured to be attached to the first lateral side by the magnetic force between the first magnet and the second magnet. According to the above-mentioned embodiment, the wearable device may provide accuracy of the information related to the user provided from the first sensor and the second sensor, by providing the structure in which the first ring and the second ring may be coupled such that the position of the second sensor relative to the first sensor is fixed. The above-mentioned embodiment may have various effects including the above-mentioned effect.

According to an embodiment, the first housing may further include a third magnet (e.g., the third magnet 312 of FIG. 3A) disposed on the first lateral side and facing the first magnet. The second housing may further include a fourth magnet (e.g., the fourth magnet 322 of FIG. 3B) disposed on the second lateral side and facing the second magnet. A position of the second sensor relative to the first sensor may be configured to be fixed by the magnetic force between the first magnet and the second magnet and magnetic force between the third magnet and the fourth magnet. According to the above-mentioned embodiment, the wearable device may provide accuracy of the information related to the user provided from the first sensor and the second sensor, by providing the structure in which the first ring and the second ring may be coupled such that the position of the second sensor relative to the first sensor is fixed. The above-mentioned embodiment may have various effects including the above-mentioned effect.

According to an embodiment, the second sensor may face the first sensor while the first ring is coupled with the second ring. According to the above-mentioned embodiment, the wearable device may provide accuracy of the information related to the user provided from the first sensor and the second sensor, by providing the structure in which the first ring and the second ring may be coupled such that the position of the second sensor relative to the first sensor is fixed. The above-mentioned embodiment may have various effects including the above-mentioned effect.

According to an embodiment, the first housing may include a first hole (e.g., the first hole 315 of FIG. 3A) for passing the portion of the body of the user. The second housing may include a second hole (e.g., the second hole 325 of FIG. 3B) configured to pass the portion of the body of the user by being connected with the first hole while the first ring is coupled with the second ring. The first magnet may include a first set of magnets (e.g., the first set of magnets 610 of FIG. 6B) spaced apart from each other at a designated interval along the first hole. The second ring may be configured to be rotatable relative to the first ring via magnetic force between the first set of magnets and the second magnet while coupled with the first ring. According to the above-mentioned embodiment, the wearable device may provide various user experiences to the user by including the second ring coupled to the first ring so as to be rotatable relative to the first ring. The above-mentioned embodiment may have various effects including the above-mentioned effect.

According to an embodiment, the wearable device may further comprise at least one processor (e.g., the at least one processor 301 of FIG. 3A), a Hall sensor (e.g., the Hall sensor 303 of FIG. 3A) configured to sense the magnetic force between the first magnet and the second magnet, and communication circuitry (e.g., the communication circuitry 302 of FIG. 3A) for communication with an external electronic device. The at least one processor may be configured to identify a coupling of the first ring and the second ring through the Hall sensor. The at least one processor may be configured to, based on identifying the coupling of the first ring and the second ring, identify, via the magnetic force between the first set of magnets and the second magnet, a rotation of the second ring relative to the first ring. The at least one processor may be configured to, based on identifying the rotation of the second ring relative to the first ring, control, via the communication circuitry, the external electronic device. According to the above-mentioned embodiment, the wearable device may provide various user experiences to the user by including the second ring coupled to the first ring so as to be rotatable relative to the first ring. The above-mentioned embodiment may have various effects including the above-mentioned effect.

According to an embodiment, the second sensor may further include another light emitting portion (e.g., the second light emitting portion 361 of FIG. 3B) spaced apart from the light receiving portion and configured to emit light toward the portion of the body of the user. The first sensor may further include another light receiving portion (e.g., the first light receiving portion 352 of FIG. 3A) configured to receive, via the second housing coupled to the first housing, at least a portion of the light being emitted from the other light emitting portion and passing through the portion of the body of the user. According to the above-mentioned embodiment, the wearable device may provide accuracy of the information related to the user provided from the first sensor and the second sensor, by providing the structure in which the first ring and the second ring may be coupled such that the position of the second sensor relative to the first sensor is fixed. The above-mentioned embodiment may have various effects including the above-mentioned effect.

According to an embodiment, the wearable device may further comprise at least one processor, a Hall sensor configured to sense the magnetic force between the first magnet and the second magnet, and communication circuitry for communication with an external electronic device. The at least one processor may be configured to identify a coupling of the first ring and the second ring through the Hall sensor. The at least one processor may be configured to, based on identifying the coupling of the first ring and the second ring, identify, through the first sensor, a strength of a first signal indicating information related to the user, received by the other light receiving portion. The at least one processor may be configured to identify, through the second sensor, a strength of a second signal indicating information related to the user, received by the light receiving portion. The at least one processor may be configured to provide, via the communication circuitry, information indicating the strength of the first signal and the strength of the second signal using the external electronic device. According to the above-mentioned embodiment, the wearable device may provide various user experiences to the user by providing information related to the first signal and the second signal via the external electronic device. The above-mentioned embodiment may have various effects including the above-mentioned effect.

According to an embodiment, the wearable device may further comprise at least one processor, a Hall sensor configured to sense the magnetic force between the first magnet and the second magnet, and communication circuitry for communication with an external electronic device. The at least one processor may be configured to identify whether the first ring and the second ring are coupled through the Hall sensor. The at least one processor may be configured to, based on identifying that the first ring and the second ring are coupled, provide, via the external electronic device connected with the wearable device by the communication circuitry, information related to the first ring and the second ring. According to the above-mentioned embodiment, the wearable device may provide various user experiences to the user by providing information related to the first ring and the second ring via the external electronic device. The above-mentioned embodiment may have various effects including the above-mentioned effect.

According to an embodiment, the wearable device may further comprise power management circuitry (e.g., the power management circuitry 305 of FIG. 3A). The at least one processor may be configured to identify a user input received by the external electronic device connected with the wearable device via the communication circuitry. The at least one processor may be configured to, based on identifying the user input, control, via the power management circuitry, power supply to at least a portion of electronic components in the first housing including the first sensor and at least a portion of electronic components in the second housing including the second sensor. According to the above-mentioned embodiment, the wearable device may provide various user experiences to the user by being configured to control electronic components in the first ring and electronic components in the second ring via the external electronic device. The above-mentioned embodiment may have various effects including the above-mentioned effect.

According to an embodiment, the at least one processor may be configured to bypass providing at least a portion of information related to the first ring and the second ring via the external electronic device, based on identifying that the first ring and the second ring are separated. According to the above-mentioned embodiment, the wearable device may provide various user experiences to the user by providing information related to the first ring and the second ring via the external electronic device. The above-mentioned embodiment may have various effects including the above-mentioned effect.

According to an embodiment, the wearable device may further comprise a third ring (e.g., the third ring 203 of FIG. 6A) disposed between the first ring and the second ring while the first ring and the second ring are coupled. The first ring and the second ring may be each rotatable relative to the third ring. According to the above-mentioned embodiment, the wearable device may provide various user experiences to the user by including the third ring. The above-mentioned embodiment may have various effects including the above-mentioned effect.

According to an embodiment, the second housing may further include at least one conductive pin (e.g., the at least one conductive pin 810 of FIG. 8). The first housing may further include at least one connector hole (e.g., the at least one connector hole 820 of FIG. 8) electrically connected with the at least one conductive pin while the first ring is coupled with the second ring. According to the above-mentioned embodiment, the wearable device may be configured such that electronic components in the wearable device are electrically connected to each other by including the at least one conductive pin and the at least one connector hole. The above-mentioned embodiment may have various effects including the above-mentioned effect.

According to an embodiment, the first ring may further include a first memory (e.g., the first memory 306a of FIG. 3A) in the first housing. The second ring may further include a second memory (e.g., the second memory 306b of FIG. 3B) in the second housing. The first ring may be configured to transmit data from the first memory to the second memory or receive data from the second memory to the first memory via the at least one conductive pin connected with the at least one connector hole. According to the above-mentioned embodiment, the first ring may provide various user experiences to the user by being electrically connected with the second ring. The above-mentioned embodiment may have various effects including the above-mentioned effect.

According to an embodiment, the first ring may further include a first battery (e.g., the first battery 304a of FIG. 3A) for supplying power to the first ring. The second ring may further include a second battery (e.g., the second battery 304b of FIG. 3B) for supplying power to the second ring. The first ring may be configured to supply power from the first battery to the second battery or receive power from the second battery to the first battery via the at least one conductive pin connected with the at least one connector hole. According to the above-mentioned embodiment, the first ring may provide various user experiences to the user by being electrically connected with the second ring. The above-mentioned embodiment may have various effects including the above-mentioned effect.

According to an embodiment, a wearable device may comprise a first ring including a first housing including a first magnet, and electronic components including a first sensor in the first housing. The wearable device may comprise a second ring including a second housing including a second magnet, and electronic components including a second sensor in the second housing, the second ring being detachably coupled with the first ring by magnetic force between the first magnet and the second magnet. The wearable device may comprise at least one processor, a Hall sensor configured to sense the magnetic force between the first magnet and the second magnet, and communication circuitry for communication with an external electronic device. The first sensor may include a light emitting portion configured to emit light toward a portion of a body of a user on which the wearable device is worn. The second sensor may include a light receiving portion configured to receive, via the second housing coupled to the first housing, at least a portion of the light being emitted from the light emitting portion and passing through the portion of the body of the user. The at least one processor may be configured to identify whether the first ring and the second ring are coupled through the Hall sensor. The at least one processor may be configured to, based on identifying that the first ring and the second ring are coupled, provide information related to the first ring and the second ring via the external electronic device connected with the wearable device by the communication circuitry. According to the above-mentioned embodiment, the wearable device may provide accuracy of information related to the user provided from the first sensor and the second sensor, by providing a structure in which the first ring and the second ring may be coupled such that a position of the second sensor relative to the first sensor is fixed. The above-mentioned embodiment may have various effects including the above-mentioned effect.

According to an embodiment, the second sensor may face the first sensor while the first ring is coupled with the second ring. According to the above-mentioned embodiment, the wearable device may provide accuracy of the information related to the user provided from the first sensor and the second sensor, by providing the structure in which the first ring and the second ring may be coupled such that the position of the second sensor relative to the first sensor is fixed. The above-mentioned embodiment may have various effects including the above-mentioned effect.

According to an embodiment, the first housing may include a first hole for passing the portion of the body of the user. The second housing may include a second hole configured to pass the portion of the body of the user by being connected with the first hole while the first ring is coupled with the second ring. The first magnet may include a first set of magnets spaced apart from each other at a designated interval along the first hole. The second ring may be configured to be rotatable relative to the first ring via magnetic force between the first set of magnets and the second magnet while coupled with the first ring. According to the above-mentioned embodiment, the wearable device may provide various user experiences to the user by including the second ring coupled to the first ring so as to be rotatable relative to the first ring. The above-mentioned embodiment may have various effects including the above-mentioned effect.

According to an embodiment, the second sensor may further include another light emitting portion spaced apart from the light receiving portion and configured to emit light toward the portion of the body of the user. The first sensor may further include another light receiving portion configured to receive, via the second housing coupled to the first housing, at least a portion of the light being emitted from the other light emitting portion and passing through the portion of the body of the user. According to the above-mentioned embodiment, the wearable device may provide accuracy of the information related to the user provided from the first sensor and the second sensor, by providing the structure in which the first ring and the second ring may be coupled such that the position of the second sensor relative to the first sensor is fixed. The above-mentioned embodiment may have various effects including the above-mentioned effect.

According to an embodiment, the wearable device may further comprise power management circuitry. The at least one processor may be configured to identify a user input received by the external electronic device connected with the wearable device via the communication circuitry. The at least one processor may be configured to, based on identifying the user input, control, via the power management circuitry, power supply to at least a portion of the electronic components and at least a portion of the electronic components. According to the above-mentioned embodiment, the wearable device may provide various user experiences to the user by being configured to control electronic components in the first ring and electronic components in the second ring via the external electronic device. The above-mentioned embodiment may have various effects including the above-mentioned effect.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device (200), comprising:
a first ring (201) including:
a first housing (310) including a first magnet (311), and
a first sensor (350) in the first housing (310); and
a second ring (202) including:
a second housing (320) including a second magnet (321), and
a second sensor (360) in the second housing (320), the second ring (202) being detachably coupled with the first ring (201) by magnetic force between the first magnet (311) and the second magnet (321),
wherein the first sensor (350) includes: a light emitting portion (351) configured to emit light toward a portion (20) of a body of a user on which the wearable device (200) is worn, and
wherein the second sensor (360) includes: a light receiving portion (362) configured to receive, via the second housing (320) coupled to the first housing (310), at least a portion of the light being emitted from the light emitting portion (351) and passing through the portion (20) of the body of the user.

2. The wearable device (200) of claim 1,
wherein the first housing (310) further includes a first lateral side (310c) on which the first magnet (311) is disposed, and
wherein the second housing (320) further includes a second lateral side (320c) on which the second magnet (321) is disposed, the second lateral side (320c) being configured to be attached to the first lateral side (310c) by the magnetic force between the first magnet (311) and the second magnet (321).

3. The wearable device (200) of claim 1 or 2,
wherein the first housing (310) further includes a third magnet (312) disposed on the first lateral side (310c) and facing the first magnet (311),
wherein the second housing (320) further includes a fourth magnet (322) disposed on the second lateral side (320c) and facing the second magnet (321), and
wherein a position of the second sensor (360) relative to the first sensor (350) is configured to be fixed by the magnetic force between the first magnet (311) and the second magnet (321) and magnetic force between the third magnet (312) and the fourth magnet (322).

4. The wearable device (200) of any one of claims 1 to 3, wherein the second sensor (360) faces the first sensor (350) while the first ring (201) is coupled with the second ring (202).

5. The wearable device (200) of any one of claims 1 to 4,
wherein the first housing (310) further includes a first hole (315) for passing the portion (20) of the body of the user,
wherein the second housing (320) further includes a second hole (325) configured to pass the portion (20) of the body of the user by being connected with the first hole (315) while the first ring (201) is coupled with the second ring (202),
wherein the first magnet (311) includes a first set of magnets (610) spaced apart from each other at a designated interval along the first hole (315), and
wherein the second ring (202) is configured to be rotatable relative to the first ring (201) via magnetic force between the first set of magnets (610) and the second magnet (321) while coupled with the first ring (201).

6. The wearable device (200) of any one of claims 1 to 5, further comprising:
at least one processor (301);
a Hall sensor (303) configured to sense the magnetic force between the first magnet (311) and the second magnet (321); and
communication circuitry (302) for communication with an external electronic device (101),
wherein the at least one processor (301) is configured to:
identify a coupling of the first ring (201) and the second ring (202) through the Hall sensor (303),
based on identifying the coupling of the first ring (201) and the second ring (202), identify, via the magnetic force between the first set of magnets (610) and the second magnet (321), a rotation of the second ring (202) relative to the first ring (201), and
based on identifying the rotation of the second ring (202) relative to the first ring (201), control, via the communication circuitry (302), the external electronic device (101).

7. The wearable device (200) of any one of claims 1 to 6,
wherein the second sensor (360) further includes another light emitting portion (361) spaced apart from the light receiving portion (362) and configured to emit light toward the portion (20) of the body of the user; and
wherein the first sensor (350) further includes another light receiving portion (352) configured to receive, via the second housing (320) coupled to the first housing (310), at least a portion of the light being emitted from the other light emitting portion (361) and passing through the portion (20) of the body of the user.

8. The wearable device (200) of any one of claims 1 to 7, further comprising:
at least one processor (301),
a Hall sensor (303) configured to sense the magnetic force between the first magnet (311) and the second magnet (321), and
communication circuitry (302) for communication with an external electronic device (101),
wherein the at least one processor (301) is configured to:
identify a coupling of the first ring (201) and the second ring (202) through the Hall sensor (303),
based on identifying the coupling of the first ring (201) and the second ring (202), identify, through the first sensor (350), a strength (s1) of a first signal indicating information related to the user, received by the other light receiving portion (352),
identify, through the second sensor (360), a strength (s2) of a second signal indicating information related to the user, received by the light receiving portion (362), and
provide, via the communication circuitry (302), information indicating the strength (s1) of the first signal and the strength (s2) of the second signal using the external electronic device (101).

9. The wearable device (200) of any one of claims 1 to 8, further comprising:
at least one processor (301),
a Hall sensor (303) configured to sense the magnetic force between the first magnet (311) and the second magnet (321), and
communication circuitry (302) for communication with an external electronic device (101),
wherein the at least one processor (301) is configured to:
identify whether the first ring (201) and the second ring (202) are coupled through the Hall sensor (303), and
based on identifying that the first ring (201) and the second ring (202) are coupled, provide, via the external electronic device (101) connected with the wearable device (200) by the communication circuitry (302), information related to the first ring (201) and the second ring (202).

10. The wearable device (200) of any one of claims 1 to 9, further comprising:
power management circuitry (305),
wherein the at least one processor (301) is configured to:
identify a user input received by the external electronic device (101) connected with the wearable device (200) via the communication circuitry (302), and
based on identifying the user input, control, via the power management circuitry (305), power supply to at least a portion of electronic components in the first housing (310) including the first sensor (350) and at least a portion of electronic components in the second housing (320) including the second sensor (360).

11. The wearable device (200) of any one of claims 1 to 10,
wherein the at least one processor (301) is configured to bypass providing at least a portion of information related to the first ring (201) and the second ring (202) via the external electronic device (101), based on identifying that the first ring (201) and the second ring (202) are separated.

12. The wearable device (200) of any one of claims 1 to 11, further comprising:
a third ring (203) disposed between the first ring (201) and the second ring (202) while the first ring (201) and the second ring (202) are coupled,
wherein the first ring (201) and the second ring (202) are each rotatable relative to the third ring (203).

13. The wearable device (200) of any one of claims 1 to 12,
wherein the second housing (320) further includes at least one conductive pin (810), and
wherein the first housing (310) further includes at least one connector hole (820) electrically connected with the at least one conductive pin (810) while the first ring (201) is coupled with the second ring (202).

14. The wearable device (200) of any one of claims 1 to 13,
wherein the first ring (201) further includes a first memory (306a) in the first housing (310),
wherein the second ring (202) further includes a second memory (306b) in the second housing (320), and
wherein the first ring (201) is configured to transmit data from the first memory (306a) to the second memory (306b) or receive data from the second memory (306b) to the first memory (306a) via the at least one conductive pin (810) connected with the at least one connector hole (820).

15. The wearable device (200) of any one of claims 1 to 14,
wherein the first ring (201) further includes a first battery (304a) for supplying power to the first ring (201),
wherein the second ring (202) further includes a second battery (304b) for supplying power to the second ring (202), and
wherein the first ring (201) is configured to supply power from the first battery (304a) to the second battery (304b) or receive power from the second battery (304b) to the first battery (304a) via the at least one conductive pin (810) connected with the at least one connector hole (820).
